# EUROPÄISCHE PATENTSCHRIFT

(11) **EP 1 037 568 B1**
(45) Veröffentlichungstag und Bekanntmachung des Hinweises auf die Patenterteilung: **01.10.2003**
(21) Anmeldenummer: 98963559.4
(22) Anmeldetag: 10.12.1998
(51) Int. Cl.: A61B 19/02, B65D 45/22

(54) **STERILCONTAINER FÜR MEDIZINISCHE ZWECKE**
STERILE CONTAINER FOR MEDICAL APPLICATIONS
RECIPIENT STERILE POUR APPLICATIONS MEDICALES

(30) Priorität: 13.12.1997 DE 19755532
(43) Veröffentlichungstag der Anmeldung: 27.09.2000
(73) Patentinhaber: Aesculap AG & Co. KG, 78532 Tuttlingen (DE)
(72) Erfinder: GABELE, Lorenz, D-88605 Sauldorf (DE); SCHWANKE, Wolfgang, D-78604 Rietheim-Weilheim (DE)
(74) Vertreter: Böhme, Ulrich, Dr. Dipl.-Phys.
(86) Internationale Anmeldenummer: EP9808068
(87) Internationale Veröffentlichungsnummer: WO99030630

(56) Entgegenhaltungen:
- DE-A- 4 127 893
- GB-A- 1 171 420
- GB-A- 190 901 818
- US-A- 4 331 257

## Beschreibung

Die Erfindung betrifft einen Sterilcontainer für medizinische Zwecke mit einem wannenförmigen Unterteil und einem dichtend auf dieses aufsetzbaren Deckel, der durch einen Verschluß gegen das Unterteil spannbar ist, wobei der Verschluß eine zwischen einer Offenstellung und einer Schließstellung schwenkbare Klappe mit einem Rastvorsprung, der als von der Klappe separater Rastkörper ausgebildet ist, und eine Rastnase mit einem Rücksprung zur Aufnahme des Rastvorsprungs in der Schließstellung der Klappe aufweist.

Derartige Sterilcontainer werden dadurch dichtend verschlossen, daß zwischen Deckel und Wanne eine elastisch verformbare Dichtung angeordnet wird. Spannt man den Deckel gegen die Wanne, wird diese Dichtung elastisch zusammengedrückt und stellt sicher, daß längs des gesamten Umfangs eine zuverlässige Abdichtung eintritt. Das Zusammenspannen wird durch einen speziellen Verschluß ermöglicht, der bei bekannten Sterilcontainern eine an einem Teil (Deckel oder Unterteil) schwenkbar gelagerte Klappe und eine am jeweils anderen Teil fixierte Rastnase umfaßt. Dabei ist die Rastnase insgesamt elastisch verformbar, so daß ein Rastvorsprung an der Klappe bei deren Schwenkbewegung in eine Rastausnehmung der Rastnase einrasten kann, bei der Schwenkbewegung wird die Rastnase so elastisch verformt, daß der Rastvorsprung in die Rastausnehmung eingleiten kann. Die notwendige Spannkraft zum Zusammenspannen von Dekkel und Unterteil wird durch die Elastizität der Rastnase geliefert.

Obwohl diese Konstruktion sich sehr bewährt hat und daher auch sehr verbreitet ist, ergeben sich gewisse Schwierigkeiten beim Ausgleichen von Fertigungstoleranzen und bei der Anpassung der notwendigen Spannkraft zwischen Deckel und Unterteil.

Aus der GB-A-J01818 ist ein Sterilisierbehälter bekannt, bei dem der Rastvorsprung durch einen separaten Rastkörper gebildet wird, der als Schwenkhebel an der ihrerseits verschwenkbaren Klappe gelagert ist, beim Verschließen erfolgt ein Verschwenken über einen Totpunkt hinweg, die Federwirkung wird durch die inhärente Elastizität der Klappe und des Schwenkhebels erzeugt und ist daher nur in sehr geringen Grenzen wählbar, d.h. Fertigungstoleranzen sind bei dieser Konstruktion kaum ausgleichbar.

Es ist Aufgabe der Erfindung, einen gattungsgemäßen Sterilcontainer so auszugestalten, daß es konstruktionsbedingt möglich wird, Fertigungstoleranzen besser auszugleichen und gegebenenfalls auch die Spannkraft zwischen Deckel und Unterteil besser einstellen zu können.

Diese Aufgabe wird bei einem Sterilcontainer der eingangs beschriebenen Art erfindungsgemäß dadurch gelöst, daß der Rastvorsprung in der Klappe in einer Richtung elastisch verschiebbar ist, in der er aus dem Rücksprung der Rastnase entfernt wird.

Bei der neuen Konstruktion wird also der Rastvorsprung in der Klappe seinerseits elastisch verschiebbar ausgestaltet, und entsprechend den Abmessungen der Klappe steht dafür ein relativ großer Verschiebeweg zu Verfügung, der in der Regel wesentlich größer ist, als der Verschiebeweg, den eine elastisch verformbare Rastnase bieten kann. Diese Vergrößerung des Verschiebewegs erleichtert einen Anpassung an Fertigungstoleranzen, außerdem ist es möglich, die Federkraft zu variieren, mit der der Rastvorsprung in Richtung auf den Rücksprung verschoben wird.

Grundsätzlich ist es möglich, den Rastvorsprung einstückig mit der Klappe auszubilden, die elastische Verschiebbarkeit wird dann dadurch erreicht, daß im Verbindungsbereich zwischen Rastvorsprung und Klappe elastisch verformbare Verbindungsglieder angeordnet sind, beispielsweise federförmige Stege oder dergleichen.

Bei einer besonders bevorzugten Ausführungsform ist jedoch vorgesehen, daß der Rastvorsprung als von der Klappe separater Rastkörper ausgebildet ist, der in der Klappe in einer Führung verschiebbar gelagert ist. Eine solche Ausgestaltung hat den Vorteil einer besonders einfachen Herstellbarkeit, außerdem können unterschiedliche Materialien verwendet werden, beispielsweise kann der Rastvorsprung aus einem sterilisierbaren Kunststoff bestehen, die Klappe dagegen aus Metall.

Günstig ist es, wenn die Führung an einem Ende offen und am anderen Ende verschlossen ist. Dies ermöglicht eine besonders leichte Einsetzbarkeit des Rastkörpers, der einfach von der offenen Seite in die Führung eingeschoben wird. Dieses offene Ende kann vorzugsweise der Schwenkachse der Klappe zugewandt sein.

Eine besonders einfach herstellbare Führung ergibt sich, wenn diese durch die Randstreifen eines Ausschnitts in der Klappe gebildet wird, die in seitliche Längsnuten des Rastkörpers eingreifen.

Es ist weiterhin vorteilhaft, wenn der Rastkörper in der Führung durch einen Anschlag unverlierbar gesichert ist, der beim Einschieben des Rastkörpers in die Führung elastisch außer Eingriff bewegbar ist. Insbesondere kann ein solcher Anschlag als elastische Raste ausgebildet sein, die in eine parallel zur Führung verlaufende Ausnehmung eintaucht.

Dadurch ergibt sich eine besonders einfache Montierbarkeit, es genügt nämlich, den Rastkörper von der offenen Seite her in die Führung einzuschieben, dabei wird der Anschlag elastisch außer Eingriff gebracht, bis der Rastkörper in die Führung eingeschoben ist. Dann bewegt sich der Anschlag wieder in die Ruhestellung zurück und verhindert von diesem Augenblick an ein Herausziehen des Rastkörpers aus der Führung.

Zwischen dem Rastkörper und der Klappe sind vorzugsweise Federmittel angeordnet, die die elastische Verschiebung des Rastkörpers bewirken.

Es kann sich dabei um herkömmliche Federmittel handeln, beispielsweise um Schraubenfedern.

Günstig ist es dabei, wenn die Klappe und/oder der Rastkörper einen Aufnahmeraum für die Federmittel aufweisen, so daß diese nur lose zwischen Klappe und Rastkörper eingesetzt werden und dann in dem Aufnahmeraum gehalten werden, zusätzliche Haltemittel sind dann überflüssig.

Bei einer besonders bevorzugten Ausführungsform ist vorgesehen, daß der Aufnahmeraum so dimensioniert ist, daß er als Federmittel mehrere Druckfedern nebeneinander aufnehmen kann. Dies ermöglicht es durch das Einsetzen einer unterschiedlichen Anzahl von derartigen Druckfedern die elastischen Kräfte zu verändern, mit denen der Rastkörper in Richtung auf den Rücksprung der Rastnase verschoben wird. Damit können die Spannkräfte eingestellt werden, die ein solcher Verschluß ausübt. Dies ist von großer Bedeutung, da die Spannkräfte zur Erzielung einer gleichen Verformung der Dichtung zwischen Deckel und Unterteil proportional mit der Länge der Dichtung zunehmen, bei unterschiedlicher Behältergröße werden also unterschiedliche Spannkräfte benötigt. Diese lassen sich einfach durch die Wahl der Zahl der Druckfedern einstellen, und auf diese Weise kann dieselbe Verschlußkonstruktion für Behälter unterschiedlicher Größe eingesetzt werden. Durch die entsprechende Wahl der Federkraft ist es außerdem möglich, die Spannkraft an spezielle Dichtcharakteristiken der Dichtung anzupassen, bei weichen Dichtungen wird die Spannkraft der Druckfedern geringer gewählt als bei harten Dichtungen.

Besonders vorteilhaft ist es, wenn die Federmittel in der entspannten Endstellung vorgespannt sind. Dies ermöglicht es, durch entsprechende Wahl der Federcharakteristiken der Druckfedern eine über die gesamte Verschiebestrecke des Rastkörpers im wesentlichen unveränderte elastische Rückstellkraft zu erzeugen, so daß diese Rückstellkräfte auch bei baubedingten Abmessungsunterschieden weitgehend gleich bleiben.

Eine besonders platzsparende und günstige Konstruktion ergibt sich, wenn der Rastkörper in der Ebene der Klappe in Richtung auf deren Schwenkachse verschiebbar gelagert ist.

Dabei kann die Klappe insbesondere U-förmig ausgebildet sein mit zwei Schenkeln, die an ihrem freien Ende die Schwenkachse definieren, und mit einem zentralen Ausschnitt, der den Rastkörper aufnimmt.

Durch die beschriebene Konstruktion der Klappe mit einem elastisch verschiebbaren Rastvorsprung ist es grundsätzlich nicht notwendig, die Rastnase ihrerseits elastisch auszubilden, es kann aber bei einer bevorzugten Ausführungsform vorgesehen sein, daß die Rastnase derart federnd ausgebildet ist, daß sie beim Verschwenken der Klappe dem Rastvorsprung an der Klappe geringfügig ausweicht. Dies ist insbesondere dann von Nutzen, wenn eine möglichst geringe Verschiebebewegung des Rastkörpers gewünscht wird, beispielsweise aus Abmessungsgründen, so daß dann sowohl die Rastnase als auch der Rastvorsprung einen Teil der federnden Verschiebung der Verschlußteile übernehmen.

Die federnde Ausbildung der Verschlußteile kann so langhubig ausgebildet sein, daß der Deckel auch bei angelegtem Verschluß entgegen der federnden Schließkraft des Verschlusses so weit von dem wannenförmigen Unterteil entfernbar ist, daß zwischen Deckel und wannenförmigem Unterteil eine Öffnung entsteht. Diese Ausgestaltung hat den Vorteil, daß damit der Deckel federnd von der Wanne abgehoben werden kann, so daß ein im Innenraum herrschender Überdruck abgebaut werden kann. Bei geeigneter Wahl der federnden Schließkräfte kann somit beispielsweise beim Sterilisiervorgang bereits die Luftentfernung aus dem Container erfolgen, es ist nicht notwendig, dazu ein eigenes Auslaßventil oder eine eigene Auslaßöffnung für die Luft im Container vorzusehen. Auch in der Phase der Nachtrocknung, die in gebräuchlichen Dampfsterilisatoren als Vakuumphase ausgebildet ist, läßt sich das Abheben des Deckels nutzen, um den Containerinhalt zu trocknen. Die feuchtigkeitsgesättigte Atmosphäre wird dabei aus dem Containerinnenraum durch die entstehende Öffnung zwischen Wanne und Deckel abgesaugt.

Günstig ist es, wenn der Sterilcontainer bei dichtend aufgesetztem Deckel vollständig geschlossen ist und sein Innenraum nur über ein Einlaßventil mit der Umgebung in Verbindung steht. Dieses Einlaßventil kann federbelastet sein. Ein Auslaßventil ist bei einer solchen Konstruktion jedoch nicht mehr notwendig, da die Funktion des Auslaßventils durch den federnd anhebbaren Deckel übernommen wird.

Es kann weiterhin vorgesehen sein, daß am Deckel ein Hubelement angeordnet ist, an dem der Deckel gegen die Wirkung der federnden Schließkraft des angelegten Verschlusses von der Wanne entfernbar ist. Dies ist dann von Bedeutung, wenn - beispielsweise bei der Trocknungsphase - das Anheben des Deckels nicht nur aufgrund des Überdruckes im Inneren des Behälters erfolgen soll, sondern gezielt auch von außen her, beispielsweise durch eine gesteuerte Hubvorrichtung.

Die nachfolgende Beschreibung bevorzugter Ausführungsformen der Erfindung dient im Zusammenhang mit der Zeichnung der näheren Erläuterung. Es zeigen:

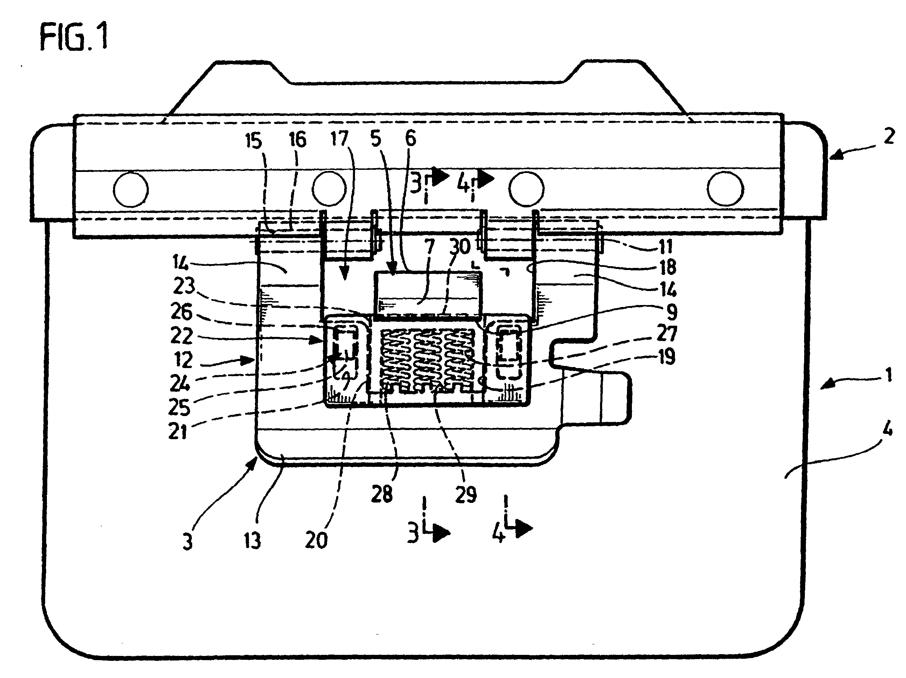

- Figur 1:: eine Seitenansicht eines verschlossenen Sterilbehälters mit einer Verschlußklappe mit elastisch verschiebbarem Rastkörper;

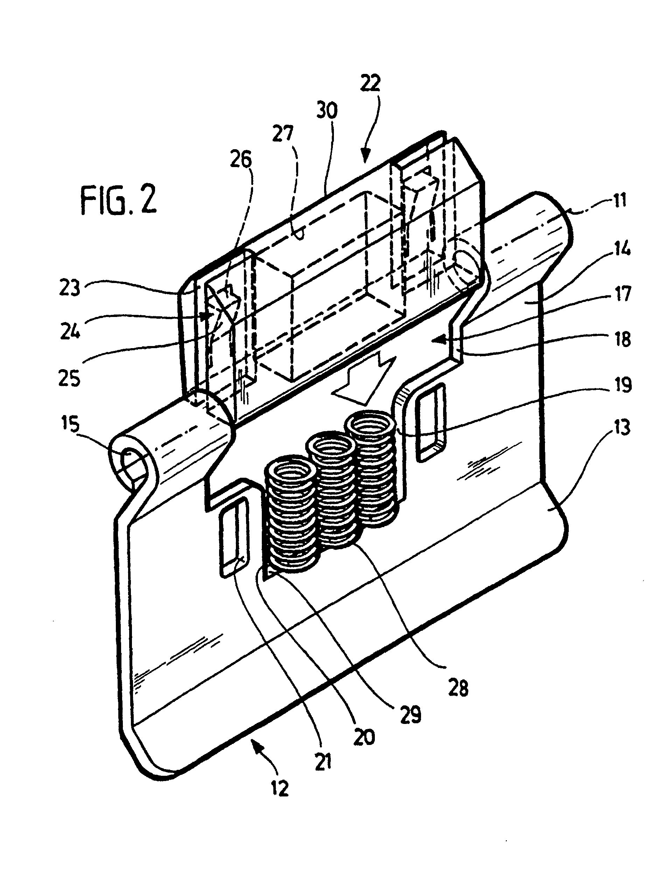

- Figur 2:: eine perspektivische Ansicht der Verschlußklappe des Containers der Figur 1 vor dem Einsetzen des Rastkörpers;

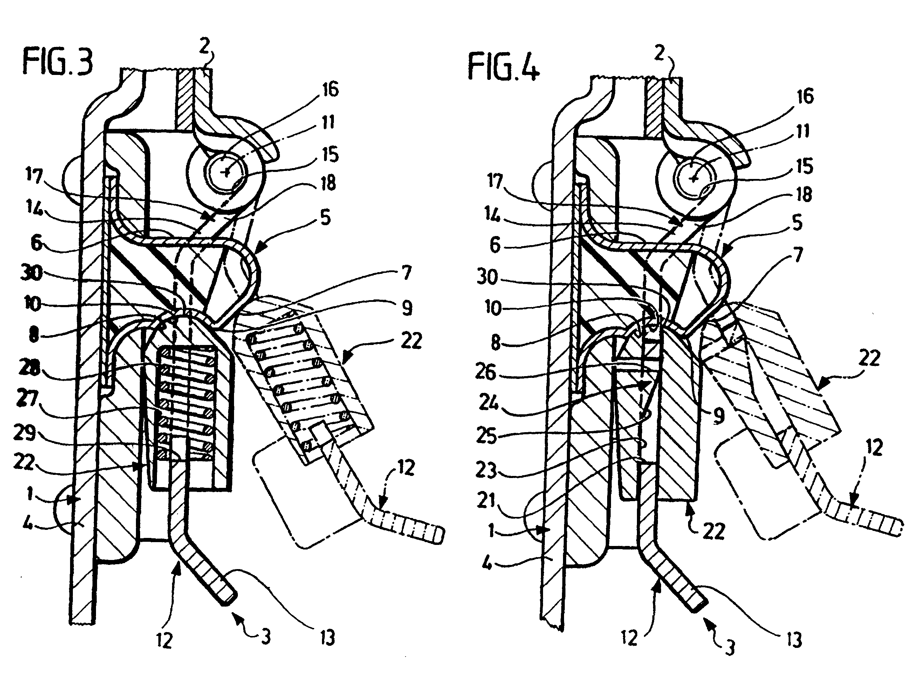

- Figur 3:: eine Schnittansicht längs Linie 3-3 in Figur 1 mit der Verschlußklappe in geschlossener Stellung (ausgezogene Linien) und in geöffneter Stellung (strichpunktierte Linien) und
- Figur 4:: eine Ansicht längs Linie 4-4 in Figur 1 mit der Verschlußklappe in geschlossener Stellung (ausgezogene Linien) und in geöffneter Stellung (strichpunktierte Linien).

Der in der Zeichnung dargestellte Sterilcontainer ist im wesentlichen quaderförmig ausgebildet und umfaßt ein wannenförmiges Unterteil 1 und einen auf dieses aufgesetzten, ebenfalls wannenförmigen Deckel 2. Zwischen Unterteil 1 und Deckel 2 ist eine umlaufende, in der Zeichnung nicht dargestellte Ringdichtung angeordnet, die den Innenraum des Behälters nach außen hin abdichtet, wenn der Deckel 2 gegen das Unterteil 1 gespannt wird.

An einander gegenüberliegenden Seitenwänden des Unterteils 1 sind gleich ausgebildete Verschlüsse 3 angeordnet, von denen nachstehend nur einer ausführlich beschrieben wird. Der Verschluß 3 umfaßt eine an der Seitenwand 4 des Unterteils 1 festgelegte Rastnase 5, die in dem dargestellten Ausführungsbeispiel aus einem dünnen, federnden Metallstreifen gebogen ist. Diese Rastnase weist eine im wesentlichen senkrecht von der Seitenwand 4 abstehende Oberseite 6 auf, die an dem der Seitenwand 4 abgewandten freien Ende der Rastnase 5 bogenförmig übergeht in eine schräg nach unten verlaufende Aufgleitfläche 7, an die sich ein bogenförmig nach innen verformter Abschnitt 8 anschließt. Im Übergangsbereich zwischen der Aufgleitfläche 7 und dem Abschnitt 8 bildet die Rastnase 5 einen nach unten weisenden Rastvorsprung 9 aus, an den sich ein durch den Abschnitt 8 gebildeter Rücksprung 10 anschließt.

Durch die Verwendung des federnden metallischen Materials ist die Rastnase 5 insgesamt geringfügig elastisch verformbar, so daß auch der Rastvorsprung 9 geringfügig elastisch nach oben verschoben werden kann.

An der Unterkante des Deckels 2 ist um eine parallel zum Deckelrand verlaufende Schwenkachse 11 eine plattenförmige Klappe 12 verschwenkbar gelagert, deren der Schwenkachse 11 gegenüberliegender Randabschnitt 13 von der Seitenwand 4 weggebogen ist, so daß das Ergreifen der Klappe 12 an ihrem freien Ende erleichtert wird.

Die Klappe 12 ist im wesentlichen U-förmig ausgebildet, wobei die beiden seitlichen Randabschnitte Arme 14 ausbilden, die an ihrem freien Ende zu einer Lageröse 15 umgebogen sind, die eine die Schwenkachse 11 definierende Lagerwelle 16 aufnehmen. Diese ist ihrerseits durch geeignete Lagerösen am Deckel 2 gelagert.

Zwischen den beiden Armen 14 befindet sich in der Klappe 12 ein Ausschnitt 17 mit einem an die Schwenkachse 11 anschließenden breiteren Bereich 18 und einem von der Schwenkachse 11 entfernteren schmaleren Bereich 19. Parallel zu den Seitenkante 20 des schmaleren Bereichs 19 verlaufen in der Klappe Langlöcher 21.

In den Ausschnitt 17 ist ein im wesentlichen quaderförmiger Rastkörper 22 eingeschoben, dessen Breite dem breiteren Bereich 18 entspricht und der an seinen Seitenwänden von oben nach unten durchgehende Längsnuten 23 aufweist, in die das Material der Klappe 12 eintritt, wenn der Rastkörper 22 von der offenen Seite des Ausschnitts 17 in diesen eingeschoben wird. Das Klappenmaterial bildet somit zusammen mit den Längsnuten 23 eine Längsführung für den im Ausschnitt 17 verschiebbar gelagerten Rastkörper 22.

Im Bereich der Längsnuten 23 ist an den Rastkörper 22 eine in die Längsnuten 23 eintauchende Raste 24 angeformt, die an ihrer Unterseite eine schräge Aufgleitfläche 25 und an ihrer Oberseite eine quer zur Verschieberichtung des Rastkörpers 22 verlaufende Anschlagfläche 26 aufweist. Diese Raste 24 ist elastisch aus der Längsnut 23 herausdrückbar, dies wird durch die Eigenelastizität des Rastkörpermaterials 22 ermöglicht. Sie ist ausgerichtet mit den Längsnuten 23 in der Klappe, so daß beim Einschieben des Rastkörpers 22 in den Ausschnitt 17 die Rasten 24 zunächst durch die Anlage der Klappe an der Aufgleitfläche 25 aus den Längsnuten 23 elastisch ausgehoben werden, nach dem Einschieben des Rastkörpers aber elastisch zurückfedern und dann in die Langlöcher 21 eingreifen. Dadurch wird der Rastkörper 22 in der Klappe unverlierbar gehalten, denn bei der Bewegung des Rastkörpers in Richtung auf das offene Ende des Ausschnitts 17 stoßen die Anschlagflächen 26 der Rasten 24 gegen die Enden der Langlöcher 21 und verhindern eine weitere Bewegung des Rastkörpers.

Im Bereich zwischen den Langlöchern 21 ist in dem Rastkörper 22 eine nach unten hin offene, quaderförmige Aufnahmekammer 27 angeordnet, in die in dem dargestellten Ausführungsbeispiel drei Schraubenfedern 28 nebeneinander eingeführt werden. Diese stützen sich einerseits am Rastkörper 22 und andererseits an der Unterkante 29 des Ausschnitts 17 ab und verschieben den Rastkörper 22 damit zum offenen Ende des Ausschnitts 17. Vorzugsweise sind die Schraubenfedern 28 vorgespannt, wenn der Rastkörper 22 in den Ausschnitt 17 eingeschoben ist.

Die Aufnahmekammer 27 ist so groß ausgebildet, daß mehrere derartige Schraubenfedern 28 nebeneinander in ihr Platz finden, der Benutzer kann jedoch die Zahl der tatsächlich eingesetzten Schraubenfedern entsprechend der gewünschten Federcharakteristik variieren, das heißt es ist ohne weiteres möglich, in die Aufnahmekammer auch nur eine einzige Schraubenfeder einzusetzen.

Die Montage der Klappe gestaltet sich somit außerordentlich einfach, es genügt nämlich, in den Rastkörper die gewünschte Zahl von Schraubenfedern 28 mit der gewünschten Federcharakteristik einzusetzen und dann den Rastkörper in den Ausschnitt 17 einzuschieben, bis die Rasten 24 in die Langlöcher 21 einrasten, daran anschließend kann die Klappe in der gleichen Weise am Deckel festgelegt werden, wie dies bei herkömmlichen Klappen der Fall war.

Die Oberseite 30 des Rastkörpers 22 ist als Rastvorsprung ausgebildet und abgerundet. Ihre Lage ist bei entspannten Schraubenfedern 28 so gewählt, daß beim Verschwenken der Klappe 12 in Schließstellung die Oberseite 30 an der Aufgleitfläche 7 der Rastnase 5 zur Anlage kommt. Dadurch wird beim weiteren Verschließen der Klappe 12 der Rastkörper 22 entgegen der Wirkung der Schraubenfeder 28 in den Ausschnitt 17 eingeschoben, so daß die Oberseite 30 des Rastkörpers 22 den Rastvorsprung 9 passieren kann. Sobald dies erfolgt ist, drückt die Schraubenfeder 28 den Rastkörper 22 mit seiner Oberseite 30 in den Rücksprung 10 der Rastnase 5 hinein, so daß jetzt die Klappe 12 in der Schließstellung fixiert ist. Die Schraubenfeder 28 (oder gegebenenfalls die Schraubenfedern 28) spannen in dieser Stellung den Deckel 2 gegen das Unterteil 1, die Spannkraft hängt von der Charakteristik der Schraubenfedern 28 und von deren Zahl ab.

Zum Öffnen des Behälters muß eine gewisse Kraft überwunden werden, um den Rastkörper 22 unter Zusammendrükkung der Schraubenfedern 28 über den Rastvorsprung 9 zu bewegen, so daß dadurch eine Sicherung gegen unbeabsichtigte Öffnung des Behälters gegeben ist.

Der Federweg der Schraubenfedern 28 kann relativ groß ausgebildet sein, so daß auch möglich ist, gegen die Kraft der Schraubenfedern 28 den Deckel geringfügig vom Unterteil abzuheben. Dies kann dann von Bedeutung sein, wenn sich im Behälter ein Überdruck aufbaut, die beschriebene federnde Konstruktion des Rastkörpers 22 führt dann zu einer Sicherheitsventilwirkung, die den Abbau eines Überdrucks ermöglicht.

## Patentansprüche

1. Sterilcontainer für medizinische Zwecke mit einem wannenförmigen Unterteil (1) und einem dichtend auf dieses aufsetzbaren Deckel (2), der durch einen Verschluß (3) gegen das Unterteil (1) spannbar ist, wobei der Verschluß (3) eine zwischen einer Offenstellung und einer Schließstellung schwenkbare Klappe (12) mit einem Rastvorsprung (30), der als von der Klappe (12) separater Rastkörper ausgebildet ist, und eine Rastnase (5) mit einem Rücksprung zur Aufnahme des Rastvorsprungs (30) in der Schließstellung der Klappe (12) aufweist, **dadurch gekennzeichnet, daß** der Rastvorsprung (30) in der Klappe (12) in einer Führung (23) in einer Richtung elastisch verschiebbar ist, in der er aus dem Rücksprung (10) der Rastnase (5) entfernt wird.

2. Sterilcontainer nach Anspruch 1, **dadurch gekennzeichnet, daß** die Führung (23) an einem Ende offen und am anderen Ende verschlossen ist.

3. Sterilcontainer nach Anspruch 2, **dadurch gekennzeichnet, daß** das offene Ende der Schwenkachse (11) der Klappe (12) zugewandt ist.

4. Sterilcontainer nach einem der Ansprüche 1 bis 3, **dadurch gekennzeichnet, daß** die Führung durch die Randstreifen eines Ausschnitts (17) in der Klappe (12) gebildet wird, die in seitliche Längsnuten (23) des Rastkörpers (22) eingreifen.

5. Sterilcontainer nach einem der Ansprüche 2 bis 4, **dadurch gekennzeichnet, daß** der Rastkörper (22) in der Führung (23) durch einen Anschlag (24) unverlierbar gesichert ist, der beim Einschieben des Rastkörpers (22) in die Führung (23) elastisch außer Eingriff bewegbar ist.

6. Sterilcontainer nach Anspruch 5, **dadurch gekennzeichnet, daß** der Anschlag (24) als elastische Raste (24) ausgebildet ist, die in eine parallel zur Führung (23) verlaufende Ausnehmung (21) eintaucht.

7. Sterilcontainer nach einem der Ansprüche 1 bis 6, **dadurch gekennzeichnet, daß** zwischen Rastkörper (22) und Klappe (12) Federmittel (28) angeordnet sind.

8. Sterilcontainer nach Anspruch 7, **dadurch gekennzeichnet, daß** die Klappe (12) und/oder der Rastkörper (22) einen Aufnahmeraum (27) für die Federmittel (28) aufweisen.

9. Sterilcontainer nach Anspruch 8, **dadurch gekennzeichnet, daß** der Aufnahmeraum (27) so dimensioniert ist, daß er als Federmittel mehrere Druckfedern (28) nebeneinander aufnehmen kann.

10. Sterilcontainer nach einem der Ansprüche 7 bis 9, **dadurch gekennzeichnet, daß** die Federmittel (28) in der entspannten Endstellung vorgespannt sind.

11. Sterilcontainer nach einem der Ansprüche 1 bis 10, **dadurch gekennzeichnet, daß** der Rastkörper (22) in der Ebene der Klappe (12) in Richtung auf deren Schwenkachse (11) verschiebbar gelagert ist.

12. Sterilcontainer nach Anspruch 11, **dadurch gekennzeichnet, daß** die Klappe (12) U-förmig ausgebildet ist mit zwei Schenkeln (14), die an ihrem freien Ende die Schwenkachse (11) definieren und mit einem zentralen Ausschnitt (17), der den Rastkörper (22) aufnimmt.

13. Sterilcontainer nach einem der voranstehenden Ansprüche, **dadurch gekennzeichnet, daß** die Rastnase (5) derart federnd ausgebildet ist, daß sie beim Verschwenken der Klappe (12) dem Rastvorsprung (30) an der Klappe (12) geringfügig ausweicht.

14. Sterilcontainer für medizinische Zwecke mit einem wannenförmigen Unterteil und einem dichtend auf dieses aufsetzbaren Deckel, der durch einen anlegbaren und wieder lösbaren Verschluß federnd gegen das Unterteil spannbar ist, wenn der Verschluß angelegt ist, nach einem der voranstehenden Ansprüche, **dadurch gekennzeichnet, daß** der Deckel (2) auch bei angelegtem Verschluß (3) entgegen der federnden Schließkraft des Verschlusses (3) so weit von dem wannenförmigen Unterteil (1) entfernbar ist, daß zwischen Deckel (2) und Unterteil (1) eine Öffnung entsteht.

15. Sterilcontainer nach Anspruch 14, **dadurch gekennzeichnet, daß** er bei dichtend aufgesetztem Deckel (2) vollständig geschlossen ist und sein Innenraum nur über ein Einlaßventil mit der Umgebung in Verbindung steht.

16. Sterilcontainer nach Anspruch 14 oder 15, **dadurch gekennzeichnet, daß** am Deckel (2) ein Hubelement angeordnet ist, an dem der Deckel (2) gegen die Wirkung der federnden Schließkraft des angelegten Verschlusses (3) von dem wannenförmigen Unterteil (1) entfernbar ist.

## Claims

1. Sterile container for medical purposes having a tub-shaped lower part (1) and a lid (2) which can be sealingly mounted on the latter and can be clamped against it by a closure (3), the closure (3) comprising a flap (12), which is pivotable between an open position and a closed position and has a locking projection (30) in the form of a locking member separate from the flap (12), and comprising a locking lug (5) with a recess for accommodating the locking projection (30) when the flap (12) is in the closed position, **characterised in that** the locking projection (30) is resiliently displaceable in the flap (12) in a guide (23) in a direction in which it is moved out of the recess (10) in the locking lug (5).

2. Sterile container according to Claim 1, **characterised in that** the guide (23) is open at one end and closed at the other end.

3. Sterile container according to Claim 2, **characterised in that** the open end faces the pivot axis (11) of the flap (12).

4. Sterile container according to one of Claims 1 to 3, **characterised in that** the guide is formed by the edge strips of a cutout (17) in the flap (12), which strips engage in lateral longitudinal channels (23) in the locking member (22).

5. Sterile container according to one of Claims 2 to 4, **characterised in that** the locking member (22) is captively secured in the guide (23) by a limit stop (24) which is movable resiliently out of engagement when the locking member (22) is pushed into the guide (23).

6. Sterile container according to Claim 5, **characterised in that** the limit stop (24) is in the form of a resilient catch (24), which penetrates into an aperture (21) extending parallel to the guide (23).

7. Sterile container according to one of Claims 1 to 6, **characterised in that** spring means (28) are arranged between locking member (22) and flap (12).

8. Sterile container according to Claim 7, **characterised in that** the flap (12) and/or the locking member (22) have a receiving space (27) for the spring means (28).

9. Sterile container according to Claim 8, **characterised in that** the receiving space (27) is so dimensioned that it can accommodate as spring means a plurality of compression springs (28) next to one another.

10. Sterile container according to one of Claims 7 to 9, **characterised in that** the spring means (28) are prestressed in the relaxed end position.

11. Sterile container according to one of Claims 1 to 10, **characterised in that** the locking member (22) is mounted so as to be displaceable in the plane of the flap (12) in the direction of the pivot axis (11) thereof.

12. Sterile container according to Claim 11, **characterised in that** the flap (12) is of U-shaped design, with two limbs (14) which define the pivot axis (11) at their free end and with a central cutout (17) which accommodates the locking member (22).

13. Sterile container according to one of the preceding claims, **characterised in that** the locking lug (5) is of such a resilient design that, when the flap (12) is pivoted, it yields slightly to make way for the locking projection (30) on the flap (12).

14. Sterile container for medical purposes having a tub-shaped lower part and a lid which can be sealingly mounted on the latter and, by means of a closure capable of being applied and released again, can be resiliently clamped against the lower part when the closure is applied, according to one of the preceding claims, **characterised in that**, even when the closure (3) is applied, the lid (2) can be moved far enough away from the tub-shaped lower part (1) against the resilient closing force of the closure (3) that an opening is formed between the lid (2) and the lower part (1).

15. Sterile container according to Claim 14, **characterised in that** it is closed completely when the lid (2) is sealingly mounted, and the inside thereof is connected to the surroundings only via an inlet valve.

16. Sterile container according to Claim 14 or 15, **characterised in that** a lifting element is arranged on the lid (2), at which lifting element the lid (2) can be moved away from the tub-shaped lower part (1) against the action of the resilient closing force of the applied closure (3).

## Revendications

1. Récipient stérile pour applications médicales comprenant une partie inférieure (1) en forme de cuve et un couvercle (2) à poser de manière étanche sur celle-ci, qui peut être serré par une fermeture (3) contre la partie inférieure (1), la fermeture (3) comportant un rabat (12), susceptible de pivoter entre une position d'ouverture et une position de fermeture et muni d'une saillie à encliqueter (30) qui est conçue sous forme de corps à encliqueter séparé du rabat (12), et un taquet d'arrêt (5) muni d'un creux destiné à recevoir la saillie à encliqueter (30) dans la position de fermeture du rabat (12), **caractérisé en ce que** la saillie à encliqueter (30) dans le rabat (12) peut coulisser de manière souple dans une glissière (23) dans une direction dans laquelle ladite saillie sort du creux (10) du taquet d'arrêt (5).

2. Récipient stérile selon la revendication 1, **caractérisé en ce que** la glissière (23) est ouverte au niveau d'une extrémité et est fermée au niveau de l'autre extrémité.

3. Récipient stérile selon la revendication 2, **caractérisé en ce que** l'extrémité ouverte est orientée vers l'axe de pivotement (11) du rabat (12).

4. Récipient stérile selon l'une quelconque des revendications 1 à 3, **caractérisé en ce que** la glissière est formée par les bordures d'une découpe (17) réalisée dans le rabat (12), lesquelles entrent en prise dans des rainures longitudinales (23) latérales du corps à encliqueter (22).

5. Récipient stérile selon l'une quelconque des revendications 2 à 4, **caractérisé en ce que** le corps à encliqueter (22) est bloqué de manière imperdable dans la glissière (23) par l'intermédiaire d'une butée (24) qui, au moment de l'introduction du corps à encliqueter (22) dans la glissière (23), peut être amenée de manière souple hors de prise.

6. Récipient stérile selon la revendication 5, **caractérisé en ce que** la butée (24) est conçue sous forme d'organe d'arrêt (24) souple, qui s'engage dans un évidement (21) parallèle à la glissière (23).

7. Récipient stérile selon l'une quelconque des revendications 1 à 6, **caractérisé en ce que** des moyens à ressort (28) sont agencés entre le corps à encliqueter (22) et le rabat (12).

8. Récipient stérile selon la revendication 7, **caractérisé en ce que** le rabat (12) et/ou le corps à encliqueter (22) comportent un logement (27) pour les moyens à ressort (28).

9. Récipient stérile selon la revendication 8, **caractérisé en ce que** le logement (27) est dimensionné de telle sorte qu'il peut recevoir plusieurs ressorts de compression (28) juxtaposés, formant les moyens à ressort.

10. Récipient stérile selon l'une quelconque des revendications 7 à 9, **caractérisé en ce que** les moyens à ressort (28) sont précontraints dans la position finale détendue.

11. Récipient stérile selon l'une quelconque des revendications 1 à 10, **caractérisé en ce que** le corps à encliqueter (22) est logé dans le plan du rabat (12) de manière mobile en direction de l'axe de pivotement (11) de celui-ci.

12. Récipient stérile selon la revendication 11, **caractérisé en ce que** le rabat (12) est conçu en forme de U avec deux branches (14), qui définissent l'axe de pivotement (11) au niveau de leurs extrémités libres et avec une découpe (17) centrale destinée à recevoir le corps à encliqueter (22).

13. Récipient stérile selon l'une quelconque des revendications précédentes, **caractérisé en ce que** le taquet d'arrêt (5) est conçu de manière souple, de telle sorte que, au moment du pivotement du rabat (12), il s'écarte légèrement de la saillie à encliqueter (30) sur le rabat (12).

14. Récipient stérile pour applications médicales comprenant une partie inférieure en forme de cuve et un couvercle à poser de manière étanche sur celle-ci, qui, au moyen d'une fermeture à appuyer et à détacher à nouveau, peut être serré de manière souple contre la partie inférieure lorsque la fermeture est mise en appui, selon l'une quelconque des revendications précédentes, **caractérisé en ce que** le couvercle (2), même si la fermeture (3) est mise en appui, peut être retiré à l'encontre de la force de fermeture exercée par les ressorts de la fermeture (3) sur une distance telle qu'il se forme une ouverture entre le couvercle (2) et la partie inférieure (1).

15. Récipient stérile selon la revendication 14, **caractérisé en ce que**, lorsque le couvercle (2) est posé de manière étanche, ledit récipient est complètement fermé et son compartiment intérieur communique avec le milieu ambiant uniquement par une soupape d'admission.

16. Récipient stérile selon la revendication 14 ou 15, **caractérisé en ce que**, au niveau du couvercle (2), est agencé un élément de levage, au niveau duquel le couvercle (2) peut être retiré de la partie inférieure (1) en forme de cuve à l'encontre de la force de fermeture exercée par les ressorts de la fermeture (3) mise en appui.
